(19) Europäisches Patentamt
European Patent Office
Office européen des brevets

(11) Veröffentlichungsnummer: **0 067 352**
**B2**

(12) # NEUE EUROPÄISCHE PATENTSCHRIFT

(45) Veröffentlichungstag der neuen Patentschrift: **13.01.88**

(51) Int. Cl.⁴: **C 07 C 148/00**, C 07 C 149/40

(21) Anmeldenummer: **82104771.9**

(22) Anmeldetag: **01.06.82**

(54) **Verfahren zur Herstellung von S-Aryl-thioglycolsäuren.**

(30) Priorität: **11.06.81 DE 3123157**

(43) Veröffentlichungstag der Anmeldung: **22.12.82 Patentblatt 82/51**

(45) Bekanntmachung des Hinweises auf die Patenterteilung: **15.08.84 Patentblatt 84/33**

(45) Bekanntmachung des Hinweises auf die Entscheidung über den Einspruch: **13.01.88 Patentblatt 88/2**

(84) Benannte Vertragsstaaten: **CH DE FR GB LI**

(56) Entgegenhaltungen:
**DE-B-2 308 262**
**DE-C-194 040**
**DE-C-201 231**
**DE-C-677 845**
**DE-C-845 461**
**DE-C-859 461**

**Chemical Abstracts, Band 52, Nr. 11, 10. Juni 1958 Columbus, Ohio, USA G.M. OKSENGENDLER et al. "Thioindigoid dyes. I. Synthesis of thiophenols and S-arylthioglycolic acids" Spalte 9051 c**
**Monatshefte f. Chemie 35 (1914) S. 1037**
**W.A. Waters J. Chem. Soc. (1942) S. 266 ff.**
**T. Saegusa et al J. Org. Chem. 23 S. 544 (1968)**
**Ber. Dtsch. Chem. Ges. 23 (1890) S. 1221 und 1222 und 1226**
**Römpps Chemie - Lexikon 7. Auflage Band 5 Seite 3058**

(73) Patentinhaber: **BAYER AG, Konzernverwaltung RP Patentabteilung, D-5090 Leverkusen 1 Bayerwerk (DE)**

(72) Erfinder: **Schütze, Detlef- Ingo, Dr., Roggendorfstrasse 51, D-5000 Köln 80 (DE)**
Erfinder: **Adams, Anton, Kapellenstrasse 20, D-5200 Siegburg (DE)**

(56) Entgegenhaltungen: (Fortsetzung)
**Ber. Dtsch. Chem. Ges. 23 (1890) S. 1218**
**Ber. Dtsch. Chem. Ges. 23 (1890) S. 1226**
**Houben Weyl, Bd. IX, S. 117**
**Ber. Dtsch. Chem. Ges. 23 (1890) S. 1218**
**"Krauch-Kunz, Reaktionen der Organischen Chemie" 5. Auflage (1976), Dr. A. Hüthig Verlag Heidelberg, Seite 330**
**Ber. Dtsch. Chem. Ges. 17 (1884) S. 2650,**
**Ber. Dtsch. Chem. Ges. 20 (1887) S. 2952**
**J. Am Chem. Soc. 46 (1924) S. 1001**
**Ber. Dtsch. Chem. Ges. 23 (1890) S. 738**
**Ber. Dtsch. Chem. Ges. 75 (1943) S. 1885**
**Chem. Abs. (1925) 19 S. 827**
**J. Chem. Soc. (1944) Seiten 18 und 393**

**Die Akte enthält technische Angaben, die nach dem Eingang der Anmeldung eingereicht wurden und die nicht in dieser Patentschrift enthalten sind.**

EP 0 067 352 B2

LIBER, STOCKHOLM 1988

**Beschreibung**

Die Erfindung befrifft ein verbessertes Verfahren zur Herstellung von S-Aryl-thioglycolsäure durch Umsetzung von aromatischen Diazoniumsalzen mit Thioglycolsäure in Gegenwart von Kupfersalzen insbesondere des zweiwertigen Kupfers.

Verfahren zur Herstellung von S-Arylthioglycolsäuren durch Umsetzung von aromatischen Diazoniumsalzen mit Thioglycolsäure sind seit Jahrzehnten bekannt. Bereits in der DE-PS-194 040 wird ein Verfahren beschrieben, bei dem man aromatische Diazoniumsalze auf Thioglycolsäure einwirken läßt und das entstandene Reaktionsprodukt, die Aryl-diazomercapto-essigsäure, direkt oder nach vorhergegangener Isolierung durch Erwärmen unter Abspaltung von Sticksfoff in die S-Aryl-thioglycolsäure überführt.

In einem weiteren Verfahren, beschrieben in der DE-PS-201 231, wird die Zersetzung der Aryl-diazomercapto-essigsäure zu den S-Aryl-thioglycolsäuren durch Zusatz von Kupferpulver bei Temperaturen von 20 bis 40° C durchgeführt.

Ferner ist das Verfahren der DE-PS-201 232 bekannt, nach dem die Zersetzung zu den S-Aryl-thioglycolsäuren durch Erhitzen in alkalischer Lösung vorgenommen wird.

Außerdem ist in der CH-PS-451 921, Beispiel 1 ein Verfahren zur Herstellung von 2,3-Dichlorphenyl-thioglycolsäure beschrieben, wobei die Umsetzung des aromatischen Diazoniumselzes mit der Thioglycolsäure in alkalischer Lösung bei 0 bis 5° C durchgeführt wird.

Der Nachteil aller dieser Verfahren besteht darin, daß Ausbeuten von nur 20-50 % zu erreichen sind und die entstehenden S-Aryl-thioglycolsäuren stark verunreinigt sind. Außerdem ist eine technische Durchführung, bei der als Zwischenprodukt eine Aryl-diazomercapto-essigsäure entsteht, die sehr leicht zu sehr heftigen Zersetzungsreaktionen neigt, sehr problematisch.

Es wurde nun überraschend gefunden, daß man S-Aryl-thioglycolsäuren in sehr guten Ausbeuten und mit hohem Reinheitsgrad erhält, wenn man Aryl-diazonium-Salze mit Thioglycolsäure in Gegenwart von Kupfersalzen, insbesondere Kupfer-II-salzen, in saurem Medium umsetzt.

Das neue Verfahren zur Herstellung von S-Aryl-thioglycolsäuren der Formel

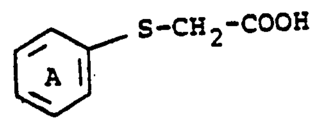

(I)

in der A einen Benzolring bezeichnet, der durch 1, 2, 3, 4 oder 5 Substituenten aus der Reihe Halogen, Nitro, Hydroxy, Mercapto, Trifluormethyl, Alkyl, Aryl, Alkoxy, Aryloxy, Acylamino, Alkylamino, Arylamino, Alkylmercapto, Arylmercapto, Cyan, Carboxy, Alkylcarbonyl, Arylcarbonyl, Alkoxycarbonyl, Aryloxycarbonyl oder gegebenenfalls substituiertes Aminocarbonyl substituiert sein kann oder für einen gegebenenfalls 1 oder 2 der vorstehend genannten Substituenten tragenden Benzolring, an den ein carbocyclischer oder heterocyclischer aromatischer Ring angegliedert ist, steht,
ist dadurch gekennzeichnet, daß man Aryl-diazoniumsalze der Formel

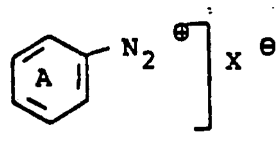

(II)

in der A die zu Formel (I) angegebene Bedeutung hat und X den Rest einer zur Salzbildung mit dem Diazoniumkation befähigten Säure bezeichnet, im wäßrig sauren Medium mit Thioglycolsäure in Gegenwart von Cu(I)- oder Cu(II)-salzen umsetzt und man das Reaktionsgemisch auf Verbindungen der Formel (I) bzw. deren Salze hin aufarbeitet.

Beispiele für erfindungsgemäß herstellbare Verbindungen der Formel (I), die einen angegliederten carbo- oder heterocyclischen Ring besitzen, sind: α- und β-Anthrachinonylthioglycolsäure, α- oder β-Naphthylthiosglycolsäure, Chinolinylthioglycolsäure.

In den Formeln (I) und (II) ist Halogen bevorzugt Chlor, Brom oder Fluor, Alkyl bevorzugt, $C_1$-$C_6$-Alkyl, Aryl bevorzugt Phenyl und Naphthyl, Alkoxy bevorzugt $C_1$-$C_6$-Alkoxy, Acyl bevorzugt ($C_1$-$C_6$-Alkyl)-carbonyl und Benzoyl; Aminocarbonyl kann beispielsweise durch $C_1$-$C_6$-Alkyl- und/oder Phenyl, Nmono oder N,N-disubstituiert sein.

Bevorzugt dient das Verfahren zur Herstellung von S-Aryl-thioglycosäuren der Formel

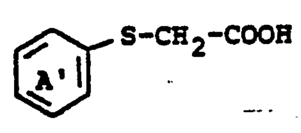

(III)

in der A' einen Benzolring bezeichnet, der durch 1, 2 oder 3 Substituenten aus der Reihe Halogen, insbesondere Chlor, Alkyl insbesondere Methyl und Ethyl, Alkoxy insbesondere Methoxy und Ethoxy, Carboxy, Nitro, Trifluormethyl substituiert sein kann oder an den ein weiterer Benzolring anelliert ist.

Besonderes Interesse verdient das neue Verfahren zur Herstellung von

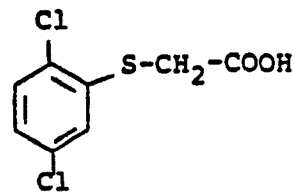

(IV)

Die zur Durchführung des Verfahrens als Ausgangsverbindungen einzusetzenden Aryl-diazoniumsalze (II) werden nach literaturbekannten Arbeitsweisen durch Diazotierung der entsprechenden Arylamine in wäßrigsaurem Milieu beispielsweise mit Natriumnitrit hergestellt. Diese Diazoniumsalz- Lösung wird dann direkt zu einem Gemisch bestehend aus Thioglycolsäure und dem Kupfersalz in Wasser zulaufen gelassen, wobei direkt unter Stickstoff-Entwicklung die Umsetzung einsetzt.

In Formel (II) steht $X^{\ominus}$ vorzugsweise für $Cl^{\ominus}$

Die Mengen und das Mischungsverhältnis an Thioglycolsäure und dem Kupfer-Salz in Wasser können je nach Art der darzustellenden S-Aryl-thioglycolsäure in weiten Bereichen variiert werden. Zum Beispiel kommen auf 1 Mol Aryl-diazoniumsalz 1 bis 1,8 Mol, vorzugsweise 1,2 bis 1,5 Mol Thioglycolsäure und 0,1 bis 1,2 Mol, vorzugsweise 0,6 bis 1,0 Mol des Kupfer-Salzes. Die Wassermenge beträgt bei den oben angegebenen Verhältnissen etwa 0,1 bis etwa 1,5 Liter.

Als Kupfersalze können beispielsweise Kupfer-(I)-chlorid, Kupfer-(II)-chlorid, Kupfer-(I)-bromid, Kupfer-(II)-bromid, Kupfer-(I)-jodid, basisches Kupfer-(II)-carbonat, Kupfer-(II)-sulfat, Kupfer-(II)-acetat, Kupfer-(II)-jodid, o. ä. zur Anwendung kommen. Bevorzugt sind die Salze des zweiwertigen Kupfers.

Die Umsetzungstemperatur beträgt vorzugsweise 0 bis 70° C, besonders bevorzugt 20 bis 30° C. Der pH-Wert des Reaktionsmediums liegt vorzugsweise zwischen 0 und 4,5, besonders bevorzugt zwischen 0,5 und 1,5. Die Reaktionszeiten liegen im allgemeinen zwischen 15 und 60 Minuten, je nach Art des umzusetzenden Aryldiazoniumsalzes. Die Aufarbeitung des Reaktionsgemisches kann auf verschiedene Arden erfolgen.

So kann man beispielsweise das saure Reaktionsgemisch auf 40 bis 50° C erhitzen und direkt die S-Aryl-thioglycolsäure isolieren. Ein anderes Verfahren besteht darin, das Reaktionsgemisch alkalisch zu stellen, wobei die S-Aryl-thioglycolsäure als Salz in Lösung geht und das Kupfer abgeschieden wird. Nach Abtrennung des Kupfers wird die S-Aryl-thioglycolsäure durch Säurezusatz gefällt und isoliert.

Man erhält auf diesem Weg die S-Aryl-thioglycolsäuren mit einem hohen Reinheitsgrad und in sehr guten Ausbeuten.

Die nach dem erfindungsgemäßen Verfahren erhältlichen S-Aryl-thioglykolsäuren sind wertvolle Zwischenprodukte beispielsweise zur Herstellung von Küpenfarbstoffen und Pigmenten.

**Beispiel 1**

a) Herstellung einer Lösung von 2,5-Dichlorphenyldiazonium-chlorid: 40,5 g 2,5-Dichloranilin werden in 64 ml 36 %-iger Salzsäure zur Bildung des Hydrochlorids verrührt. Anschließend wird mit 300 ml Wasser und 450 g Eis verdünnt und durch Zugabe einer Lösung von 18 g Natriumnitrit in 60 ml Wasser diazotiert. Nachdem 1 Stunde nachgerührt worden ist, wird das überschüssige Nitrit mit einer Lösung von 1,6 g Amidosulfonsäure in 17 ml Wasser zerstört. Diese Lösung wird anschließend umgesetzt.

b) Thioglycolsäure-Umsetzung:

In 250 ml Wasser werden 29,9 g Thioglycolsäure und 16,6 g basisches Kupfercarbonat 30 Minuten verrührt. Anschließend wird die nach a) hergestellte Diazoniumsalz-Lösung bei 20-25° C innerhalb von 15 Minuten zulaufen gelassen. Es tritt sofort unter Stickstoff-Entwicklung die Umsetzung ein. Nachdem weitere 30 Minuten nachgerührt worden sind, werden 55 ml 50 %-ige Natronlauge zugesetzt (pH 7-7,5), auf 70° C erwärmt und 1 Stunde gerührt. Danach werden weitere 20 ml 50 %-ige Natronlauge zugesetzt (pH 7,5-8), auf 95-100° C erwärmt, 30 Minuten gerührt und zur Abtrennung des Kupferhydroxids heiß filtriert. Anschließend wird das Filtrat in 85 ml 36 %-ige Salzsäure eingerührt und die ausgefallene 2,5-Dichlorphenyl-thioglycolsäure abgesaugt, mit wenig Wasser gewaschen und getrocknet. Man erhält 48,1 g 2,5-Dichlorphenyl-thioglycolsäure, d. s. 80,3 % der Theorie. Die erhaltene Säure ist 98,8 %-ig, der Schmelzpunkt beträgt 129-130° C.

**Beispiel 2**

32,2 g Thioglycolsäure und 56,4 g kristallines Kupfersulfat werden in 250 ml Wasser 30 Minuten verrührt. Anschließend wird bei 20°C eine nach Beispiel 1a) hergestellte Lösung des 2,5-Dichlorphenyldiazoniumchlorids innerhalb von 15 Minuten zulaufen gelassen. Nachdem 30 Minuten nachgerührt worden ist, werden 64 ml 36 %-ige Salzsäure zugesetzt und bei 40-50°C eine Stunde gerührt. Anschließend wird heiß abgesaugt und mit 5 %-ige Salzsäure gewaschen.

Der feuchte Nutschkuchen wird erneut in 800 ml Wasser angerührt mit 30 g Natriumcarbonat in Lösung gebracht und zur Klärung mit 7,5 g A-Kohle 15 Minuten zum Sieden erhitzt. Es wird Heiß filtriert und das Filtrat auf 85 ml 36 %-ige Salzsäure zur Fällung der 2,5-Dichlorphenylthioglycolsäure gegeben. Es wird abgesaugt, mit wenig Wasser gewaschen und getrocknet.

Man erhält 49,8 g 2,5-Dichlorphenyl-thioglycolsäure, d. s. 83,9 % der Theorie. Die Säure ist 99,8 %-ig und besitzt einen Schmelzpunkt von 129-130°C.

**Beispiel 3**

Man verrührt analog Beispiel 2, setzt jedoch statt 56,4 g Kupfersulfat 29,7 g Kupfer-I-chlorid ein.

Man erhält 44,9 g 2,5-Dichlorphenyl-thioglycolsäure, d. s. 74,6 %-ig der Theorie. Die erhaltene Säure ist 98,5 %-ig mit einem Schmelzpunkt von 120-130°C.

**Beispiel 4**

In 250 ml Wasser werden 32,2 g Thioglycolsäure und 56,4 g kristallines Kupfersulfat 30 Minuten verrührt. Anschließend wird innerhalb von 20 Minuten bei 25°C eine wäßrige Lösung von 3-Ethoxyphenyldiazoniumchlorid, hergestellt nach Beispiel 1a) aus 34,3 g 3-Ethoxy-anilin, zulaufen gelassen. Nachdem 40 Minuten nachgerührt worden ist, werden 64 ml 36 %-ige Salzsäure zugegeben. Bei 40-50°C wird 1 Stunde gerührt, heiß abgesaugt und mit 5 %-iger Salzsäure gewaschen. Der feuchte Nutschkuchen wird in 500 ml Wasser mit 30 g Natriumcarbonat gelöst und mit 5 g A-Kohle 15 Minuten zum Sieden erhitzt. Es wird heiß filtriert und das Filtrat auf 85 ml 36 %-ige Salzsäure zur Fällung der 3-Ethoxyphenyl-thioglycol-säure gegeben. Es wird abgesaugt, mit wenig Wasser gewaschen und getrocknet. Man erhält 40,4 g 3-Ethoxyphenylthioglycolsäure, d. s. 75,7 % der Theorie. Die Säure ist 99,5 %-ig mit einem Schmelzpunkt von 105-107°C.

**Beispiele 5 bis 20**

Verfährt man analog den Beispielen 1 bis 4, jedoch unter Verwendung der in der folgenden Tabelle aufgeführten aromatischen Amine, bzw. deren entsprechenden Diazoniumsalz-Lösungen, so erhält man die aufgeführten Arylthioglycolsäuren in der Regel in derselben Reinheit und in Ausbeuten von 75 % bis 85 % der Theorie.

| Bsp. Nr. | Arylamin | Aryl-thioglycolsäure |
|---|---|---|
| 5 | 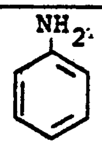 | Phenyl-thioglycolsäure |
| 6 | 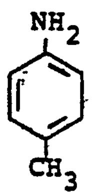 | 4-Methylphenyl-thioglycolsäure |

(Fortsetzung)

| Bsp. Nr. | Arylamin | Aryl-thioglycolsäure |
|---|---|---|
| 7 | 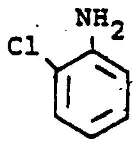 | 2-Chlorphenyl-thioglycolsäure |
| 8 | 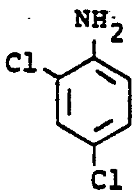 | 2,4-Dichlorphenyl-thioglycolsäure |
| 9 | 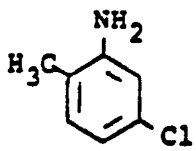 | 2-Methyl-5-chlorphenyl-thioglycolsäure |
| 10 | 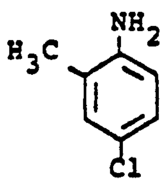 | 2-Methyl-4-chlorphenyl-thioglycolsäure |
| 11 | 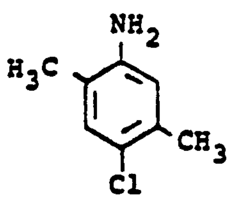 | 2,5-Dimethyl-4-chlorphenyl-thioglycolsäure |
| 12 | 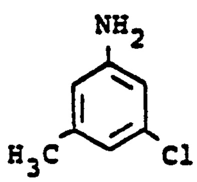 | 3-Methyl-5-chlorphenyl-thioglycolsäure |
| 13 | 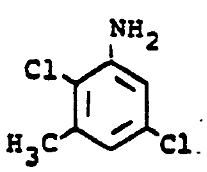 | 2,5-Dichlor-3-methylphenyl-thioglycolsäure |
| 14 | 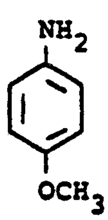 | 4-Methoxyphenyl-thioglycolsäure |

(Fortsetzung)

| Bsp. Nr. | Arylamin | Arylthioglycolsäure |
|---|---|---|
| 15 | 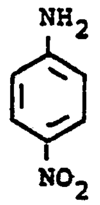 | 4-Nitrophenyl-thioglycolsäure |
| 16 | 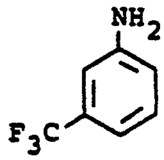 | 3-Trifluormethylphenyl-thioglycolsäure |
| 17 | 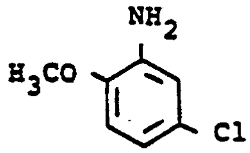 | 2-Methoxy-5-chlorphenyl-thioglycolsäure |
| 18 | 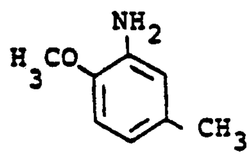 | 2-Methoxy-5-methylphenyl-thioglycolsäure |
| 19 | 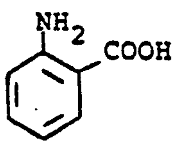 | 2-Carboxyphenyl-thioglycolsäure |
| 20 | 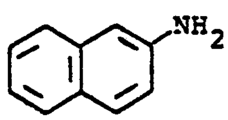 | ß-Naphthyl-thioglycolsäure |

**Patentansprüche**

1. Verfahren zur Herstellung von S-Arylthioglycolsäuren der Formel

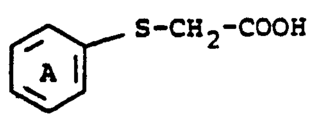

(I)

in der A einen Benzolring bezeichnet, der durch 1, 2, 3, 4 oder 5 Substituenten aus der Reihe Halogen, Nitro, Hydroxy, Mercapto, Trifluormethyl, Alkyl, Aryl, Alkoxy, Aryloxy, Acylamino, Alkylamino, Arylamino, Alkylmercapto, Arylmercapto, Cyan, Carboxy, Alkylcarbonyl, Arylcarbonyl, Alkoxycarbonyl, Aryloxycarbonyl oder gegebenenfalls substituiertes Aminocarbonyl substituiert sein kann oder für einen gegebenenfalls 1 oder 2 der vorstehend genannten Substituenten tragenden Benzolring, an den ein carbocyclischer oder heterocyclischer aromatischer Ring angegliedert ist, steht,

dadurch gekennzeichnet, daß man Aryl-diazoniumsalze der Formel

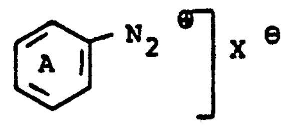

(II)

in der A die zu Formel (I) angegebene Bedeutung hat und X den Rest einer zur Salzbildung mit dem Diazoniumkation befähigten Säure bezeichnet, im wäßrig sauren Medium mit Thioglycolsäure in Gegenwart von Cu(I)- oder Cu(II)-selzen umsetzt, wobei man die Diazoniumsalz-Lösung direkt zu dem Gemisch bestehend aus Thioglycolsäure und dem Kupfersalz in Wasser zulaufen läßt, und man das Reaktionsgemisch auf Verbindungen der Formel (I) bzw. deren Salze hin aufarbeitet.

2. Verfahren gemäß Anspruch 1 zur Herstellung von S-Aryl-thioglycolsäuren der Formel

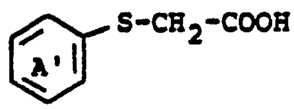

(III)

in der A' einen Benzolring bezeichnet, der durch 1, 2 oder 3 Substituenten aus der Reihe Halogen insbesondere Chlor, Alkyl insbesondere Methyl und Ethyl, Alkoxy insbesondere Methoxy und Ethoxy, Carboxy, Nitro, Trifluormethyl substituiert sein kann oder en den ein weiterer Benzolring anelliert ist.

3. Verfahren gemäß Anspruch 1 zur Herstellung von

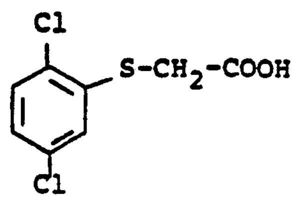

(IV)

4. Verfahren gemäß den Ansprüchen 1 bis 3, dadurch gekennzeichnet, daß man Kupfer(II)-salze einsetzt.

5. Verfahren gemäß den Ansprüchen 1 bis 3, dadurch gekennzeichnet, daß men Kupfer(II)-chlorid, Kupfer(II)-bromid, Kupfer(II)-jodid, Kupfer(II)-sulfat, Kupfer(II)-acetat oder basisches Kupfer(II)-carbonat einsetzt.

6. Verfahren gemäß den Ansprüchen 1 bis 5, dadurch gekennzeichnet, daß man bei einem pH-Wert zwischen 0 und 4,5, vorzugsweise 0,5 und 1,5 arbeitet.

7. Verfahren gemäß den Ansprüchen 1 bis 6, dadurch gekennzeichnet, daß man die Umsetzung bei 0 bis 70°C, bevorzugt 20 bis 30°C, vornimmt.

8. Verfahren gemäß den Ansprüchen 1 bis 7, dadurch gekennzeichnet, daß man pro Mol Aryldiazoniumsalz 1 bis 1,8 Mol, vorzugsweise 1,2 bis 1,5 Mol Thioglycolsäure einsetzt.

9. Verfahren gemäß den Ansprüchen 1 bis 6, dadurch gekennzeichnet, daß man pro Mol Aryldiazoniumsalz 0,1 bis 1,2 Mol, vorzugsweise 0,6 bis 1 Mol Kupfersalz einsetzt.

**Claims**

1. Process for the preparation of S-arylthioglycolic acids of the formula

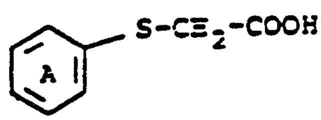

( I )

in which

A designates a benzene ring, which can be substituted by 1, 2, 3, 4 or 5 substituents from the series halogen, nitro, hydroxyl, mercapto, trifluoromethyl, alkyl, aryl, alkoxy, aryloxy, acylamino, alkylamino, arylamino, alkylmercapto, arylmercapto, cyano, carboxyl, alkylcarbonyl, arylcarbonyl, alkoxycarbonyl, aryloxycarbonyl or optionally substituted aminocarbonyl, or represents a benzene ring, which optionally carries 1 or 2 of the previously mentioned substituents, to which a carbocyclic or heterocyclic aromatic ring is attached, characterized in that aryldiazonium salts of the formula

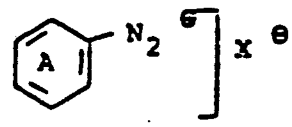

(II)

in which

A has the meanings given for the formula (I) and X designates the radical of an acid capable of forming a salt with the diazonium cation, are reacted with thioglycolic acid in an aqueous acid medium in the presence of Cu(I) or Cu(II) salts, the diazonium salt solution being directly run into the mixture consisting of thioglycolic acid and the copper, salt in water, and the reaction mixture is worked up to compounds of the formula (I) or their salts.

2. Process according to Claim 1 for the preparation of S-arylthioglycolic acids of the formula

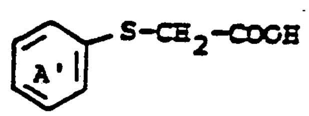

(III)

in which

A' designates a benzene ring, which can be substituted by 1, 2 or 3 substituents from the series halogen, in particular chlorine, alkyl, in particular methyl and ethyl, alkoxy, in particular methoxy and ethoxy, carboxyl, nitro or trifluoromethyl, or to which another benzene ring is fused.

3. Process according to Claim 1 for the preparation of

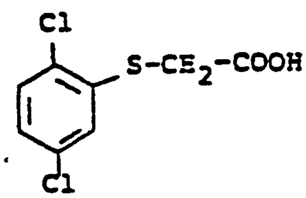

(IV)

4. Process according to Claims 1 to 3, characterized in that copper(II) salts are employed.

5. Process according to Claims 1 to 3, characterized in that copper(II) chloride, copper(II) bromide, copper(II) iodide, copper(II) sulphate, copper(II) acetate or basic copper(II) carbonate is employed.

6. Process according to Claims 1 to 5, characterized in that the reaction is carried out at a pH between 0 and 4.5, preferably 0.5 and 1.5.

7. Process according to Claims 1 to 6, characterized in that the reaction is carried out at 0 to 70°C, preferably 20 to 30°C.

8. Process according to Claims 1 to 7, characterized in that 1 to 1.8 mol, preferably 1.2 to 1.5 mol, of thioglycolic acid are employed per mol of aryldiazonium salt.

9. Process according to Claims 1 to 8, characterized in that 0.1 to 1.2 mol, preferably 0.6 to 1 mol of copper salt are employed per mol of aryldiazonium salt.

**Revendications**

1. Procédé de production d'acides S-arylthioglycoliques de formule

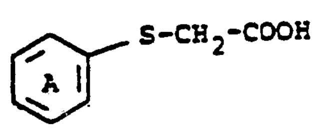

(I)

dans laquelle A désigne un noyau benzénique qui peut être substitué par 1, 2, 3, 4 ou 5 substituants de la série comprenant un halogène, un groupe nitro, hydroxy, mercapto, trifluorométhyle, alkyle, aryle, alkoxy, aryloxy, acylamino, alkylamino, arylamino, alkylmercapto, arylmercapto, cyano, carboxy, alkylcarbonyle, arylcarbonyle, alkoxycarbonyle, aryloxycarbonyle ou aminocarbonyle éventuellement substitué ou un noyau benzénique portant éventuellement un ou deux des substituants mentionnés ci-dessus, auquel est condensé un noyau aromatique carbocyclique ou hétérocylique,

caractérisé en ce qu'on fait réagir des sels d'aryldiazonium de formule

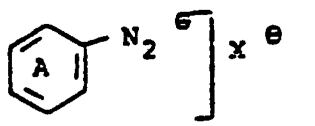

(II)

dans laquelle A a la définition indiquée pour la formule (I) et X désigne le reste d'un acide apte à former un sel avec le cation diazonium, en milieu acide aqueux avec l'acide thioglycolique en présence de sels de Cu(I) ou de Cu(II) en faisant couler la solution de sel de diazonium directement dans le mélange formé d'acide thioglycolique et du sel de cuivre dans l'eau, et on traite le mélange réactionnel en vue d'obtenir des composés de formule (I) ou leurs sels.

2. Procédé suivant la revendication 1 pour l'obtention d'acides S-arylthioglycoliques de formule

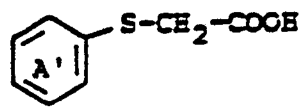

(III)

dans laquelle

A' désigne un noyau benzénique qui peut être substitué par 1, 2 ou 3 substituants de la série comprenant un halogène, notamment le chlore, un groupe alkyle, notamment les groupes méthyle et éthyle, un groupe alkoxy, notamment les groupes méthoxy et éthoxy, un groupe carboxy, nitro, trifluorométhyle ou avec lequel est condensé un autre noyau benzénique.

3. Procédé suivant la revendication 1 pour l'obtention du composé

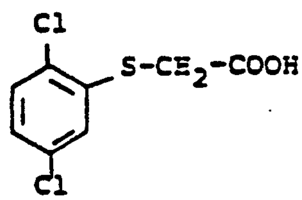

(IV)

4. Procédé suivant les revendications 1 à 3, caractérisé en ce qu'on utilise des sels de cuivre (II).

5. Procédé suivant les revendications 1 à 3, caractérisé en ce qu'on utilise du chlorure de cuivre (II), du bromure de cuivre (II), de l'iodure de cuivre (II), du sulfate de cuivre (II), de l'acétate de cuivre (II) ou du carbonate de cuivre (II) basique.

6. Procédé suivant les revendications à 5, caractérisé en ce qu'on opère à un pH compris entre 0 et 4,5, de préférence entre 0,5 et 1,5.

7. Procédé suivant les revendications 1 à 6, caractérisé en ce qu'on conduit la réaction à 0-70°C de préférence à 20-30°C.

8. Procédé suivant les revendications 1 à 7, caractérisé en ce qu'on utilise par mole de sel d'aryldiazonium 1 à 1,8 mole, de préférence 1,2 à 1,5 mole, d'acide thioglycolique.

9. Procédé suivant les revendications 1 à 8, caractérisé en ce qu'on utilise par mole de sel d'aryldiazonium 0,1 à 1,2 mole, de préférence 0,6 à 1 mole, de sel de cuivre.